# EUROPEAN PATENT APPLICATION

(11) **EP 2 933 255 A1**
(43) Date of publication of application: **21.10.2015**
(21) Application number: 14165135.6
(22) Date of filing: 17.04.2014
(51) Int. Cl.: C07D 409/10

(54) **Novel crystalline form of 1-(beta-D-glucopyranosyl)-4- methyl-3-[5-(4-fluorophenyl)-2-thienylmethyl]benzene**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to a novel crystalline form of 1-(β-D-glucopyranosyl)-4-methyl-3[5-(4-fluorophenyl)-2-thienylmethyl]benzene, to a process for its preparation and to its use for the purification of 1-(β-D-glucopyranosyl)-4-methyl-3-[5-(4-fluorophenyl)-2-thienylmethyl]benzene.

## Description

### FIELD OF THE INVENTION

The present invention relates to a novel crystalline form of 1-(β-D-glucopyranosyl)-4-methyl-3-[5-(4-fluorophenyl)-2-thienylmethyl]benzene, to a process for its preparation and to its use for the purification of 1-(β-D-glucopyranosyl)-4-methyl-3-[5-(4-fluorophenyl)-2-thienylmethyl]-benzene.

### BACKGROUND OF THE INVENTION

1-(β-D-glucopyranosyl)-4-methyl-3-[5-(4-fluorophenyl)-2-thienylmethyl]benzene, also known as canagliflozin, belongs to the therapeutic class of sodium-glucose co-transporter 2 (SGLT2) inhibitors. It received drug regulatory approval in March 2013 in the US and in November 2013 in the EU (INVOKANA™) and is indicated as an adjunct to diet and exercise to improve glycemic control in adults with type 2 diabetes mellitus. The compound 1-(β-D-glucopyranosyl)-4-methyl-3-[5-(4-fluorophenyl)-2-thienylmethyl]benzene is represented by the following general formula (I):

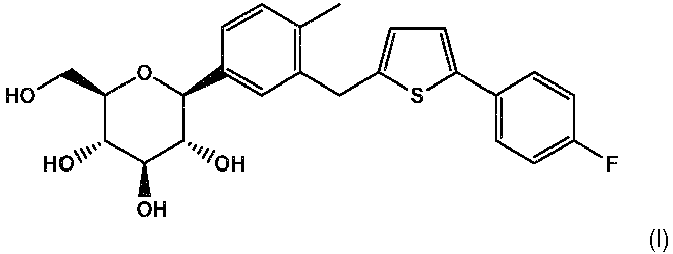

WO 2005/012326 A1 discloses 1-(β-D-glucopyranosyl)-4-methyl-3-[5-(4-fluorophenyl)-2-thienylmethyl]benzene *per se* (compound 84 on page 84). Its preparation process is generically disclosed in Example 1 of said application.

In WO 2008/069327 A1 a crystalline hemihydrate of 1-(β-D-glucopyranosyl)-4-methyl-3-[5-(4-fluorophenyl)-2-thienylmethyl]benzene as well as a process for its preparation, the process comprising the step of crystallization from a water-containing solvent, are disclosed.

WO 2009/035969 A1 discloses a crystalline form of 1-(β-D-glucopyranosyl)-4-methyl-3-[5-(4-fluorophenyl)-2-thienylmethyl]benzene. However, an overlay of the PXRDs provided in Figure 1 of WO 2009/035969 A1 and Figure 1 of WO 2008/069327 A1 shows good agreement, which confirms the presence of the same crystalline form, namely the hemihydrate, in both applications. Again, a water-containing organic solvent is used in the process for the preparation of the crystalline hemihydrate disclosed in WO 2009/035969 A1.

WO 2010/043682 A2, WO 2011/047113 A1 and WO 2012/140120 A1 disclose processes for the preparation of 1-(β-D-glucopyranosyl)-4-methyl-3-[5-(4-fluorophenyl)-2-thienylmethyl]benzene hemihydrate. In all occasions water-containing organic solvents are used in the crystallization step.

WO 2011/003976 A1 discloses a process for the preparation of crystalline 1-(β-D-glucopyranosyl)-4-methyl-3-[5-(4-fluorophenyl)-2-thienylmethyl]benzene hemihydrate having a narrow particle size distribution.

WO 2012/154812 A1 discloses co-crystals of 1-(β-D-glucopyranosyl)-4-methyl-3-[5-(4-fluorophenyl)-2-thienylmethyl]benzene) with L-proline and citric acid respectively and methods for their preparation.

WO 2013/064909 A2 discloses amorphous 1-(β-D-glucopyranosyl)-4-methyl-3-[5-(4-fluorophenyl)-2-thienylmethyl]benzene as well as co-crystals with L-proline, D-proline and L-phenylalanine respectively. Processes for the preparation of these solid forms are also disclosed.

CN103554092 A discloses a crystalline form of 1-(β-D-glucopyranosyl)-4-methyl-3-[5-(4-fluorophenyl)-2-thienylmethyl]benzene designated Form B.

CN103588762 A discloses crystalline forms of 1-(β-D-glucopyranosyl)-4-methyl-3-[5-(4-fluorophenyl)-2-thienylmethyl]benzene designated Form C and Form D.

CN103641822 A discloses a crystalline form of 1-(β-D-glucopyranosyl)-4-methyl-3-[5-(4-fluorophenyl)-2-thienylmethyl]benzene. An overlay of the PXRDs provided in Figure 1 of CN 103641822 A and Figure 1 of WO 2008/069327 A1 shows good agreement, which confirms the presence of the same crystalline form, namely the hemihydrate, in both applications.

Synthetic processes for the preparation of pharmaceutical drugs at least to some extent lead to the building of undesired chemical impurities. Limiting those impurities is essential as they may be connected with certain toxicological risks. Therefore, in order to ensure quality, safety and efficacy of the final drug product all kinds of impurities e.g. inorganic and organic impurities and in particular genotoxic impurities should be limited to the extent possible. Hence there remains a continuous need for improving the chemical purity of pharmaceutical drugs such as 1-(β-D-glucopyranosyl)-4-methyl-3-[5-(4-fluorophenyl)-2-thienylmethyl]-benzene.

### SUMMARY OF THE INVENTION

The present invention relates to a novel crystalline form of 1-(β-D-glucopyranosyl)-4-methyl-3-[5-(4-fluorophenyl)-2-thienylmethyl]benzene and to a process for its preparation. The novel form is characterized by high chemical purity and is therefore especially suitable for the purification of 1-(β-D-glucopyranosyl)-4-methyl-3-[5-(4-fluorophenyl)-2-thienylmethyl]-benzene.

In the context of the present invention the following abbreviations have the indicated meaning, unless explicitly stated otherwise:
- PXRD: powder X-ray diffraction/ diffractogram
- FTIR: Fourier transform infrared spectrum
- DSC: differential scanning calorimetry
- HPLC: high performance liquid chromatography
- RT: room temperature

### BRIEF DESCRIPTION OF THE FIGURES

Figure 1: Representative PXRD of 1-(β-D-glucopyranosyl)-4-methyl-3-[5-(4-fluorophenyl)-2-thienylmethyl]benzene Form S_{AcOH}
Figure 2: Representative FTIR spectrum of 1-(β-D-glucopyranosyl)-4-methyl-3-[5-(4-fluorophenyl)-2-thienylmethyl]benzene Form S_{AcOH}
Figure 3: Representative DSC curve of 1-(β-D-glucopyranosyl)-4-methyl-3-[5-(4-fluorophenyl)-2-thienylmethyl]benzene Form S_{AcOH}

### DETAILED DESCRIPTION OF THE INVENTION

The invention is described below in further detail by embodiments, without being limited thereto.

As used herein the term "room temperature" indicates that the applied temperature is not critical and that no exact temperature value has to be kept. Usually, "room temperature" is understood to mean temperatures of 15 to 25 °C [see e.g. European Pharmacopoeia 8.0, 1.2 (2014)].

The term "glacial acetic acid" as used herein refers to acetic acid having an assay of ≥ 99%.

The term "Form Hy0.5" as used herein refers to the crystalline 1-(β-D-glucopyranosyl)-4-methyl-3-[5-(4-fluorophenyl)-2-thienylmethyl]benzene hemihydrate disclosed in WO 2008/069327 A1. Form Hy0.5 comprises characteristic PXRD peaks at 2-Theta angles of 3.9 ± 0.2°, 13.0 ± 0.2°, 15.5 ± 0.2°, 18.8 ± 0.2° and 20.3 ± 0.2° when measured at room temperature using Cu-Kα_{1,2} radiation having a wavelength of 0.15419 nm.

The term "essentially the same" with reference to PXRD means that variabilities in peak positions and relative intensities of the peaks are to be taken into account. For example, a typical precision of the 2-Theta values is in the range of ± 0.2° 2-Theta. Thus, the diffraction peak of Form S_{AcOH} that usually appears at 18.5° 2-Theta for example can appear between 18.3° and 18.7° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

The term "essentially the same" with reference to infrared spectrometry means that variabilities in peak positions and relative intensities of the peaks are to be taken into account. For example, a typical precision of the wavenumber values is in the range of ± 2 cm⁻¹. Thus, the peak of Form S_{AcOH} that usually appears at 1693 cm⁻¹ for example can appear between 1691 and 1695 cm⁻¹ on most IR-spectrometers under standard conditions. Differences in relative intensities are typically smaller compared to X-ray diffraction. However, one skilled in the art will appreciate that small differences in peak intensities due to degree of crystallinity, sample preparation and other factors can also occur in IR-spectroscopy. Bigger intensity differences will occur when IR spectra are recorded in transmission (KBr pellet or Nujol mull) instead of attenuated total reflectance (ATR). Relative peak intensities should therefore be taken as qualitative measure only.

In a first aspect, the present invention relates to a novel crystalline form of 1-(β-D-glucopyranosyl)-4-methyl-3-[5-(4-fluorophenyl)-2-thienylmethyl]benzene, hereinafter also designated Form S_{AcOH}.

Form S_{AcOH} can be characterized by having a PXRD comprising characteristic peaks, double peaks and/or multiple peaks at 2-Theta angles of 4.8 ± 0.2°, 7.9 ± 0.2°, 14.6 ± 0.2°, 18.5 ± 0.2° and 21.5 ± 0.2° when measured at room temperature with Cu-Kα_{1,2} radiation having a wavelength of 0.15419 nm. The PXRD of Form S_{AcOH} can be further characterized by having additional characteristic peaks, double peaks and/or multiple peaks at 2-Theta angles of 6.7 ± 0.2°, 9.6±0.2°, 12.7±0.2°, 15.9±0.2°, 20.7 ± 0.2° and/or 22.4 ± 0.2°.

Alternatively, Form S_{AcOH} can be characterized by having a PXRD comprising characteristic peaks, double peaks and/or multiple peaks at 2-Theta angles of 6.7 ± 0.2°, 9.6 ± 0.2°, 18.5 ± 0.2°, 20.0 ± 0.2° and 20.7 ± 0.2° when measured at room temperature with Cu-Kα_{1,2} radiation having a wavelength of 0.15419 nm.

Alternatively, Form S_{AcOH} can be characterized by having a PXRD comprising characteristic peaks, double peaks and/or multiple peaks at 2-Theta angles of 4.8 ± 0.2°, 6.7 ± 0.2°, 7.9 ± 0.2°, 9.6 ± 0.2°, 12.7 ± 0.2°, 14.6 ± 0.2°, 15.9 ± 0.2°, 18.5 ± 0.2°, 20.7 ± 0.2° and 22.4 ± 0.2° when measured at room temperature with Cu-Kα_{1,2} radiation having a wavelength of 0.15419 nm.

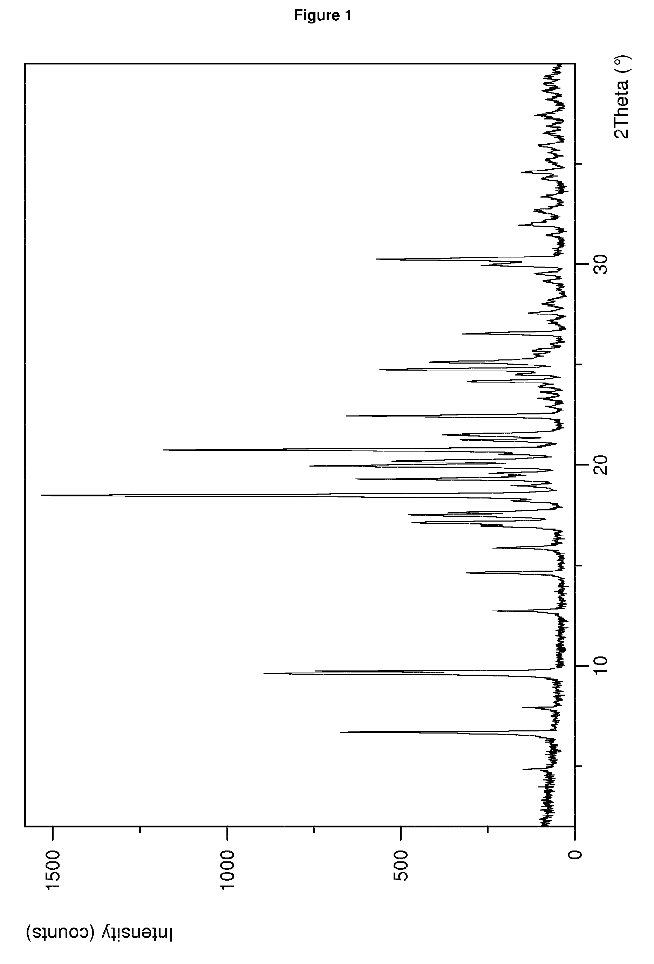

Alternatively or additionally, Form S_{AcOH} can be characterized by having a PXRD essentially the same as displayed in Figure 1 of the present invention when measured at room temperature with Cu-Kα_{1,2} radiation having a wavelength of 0.15419 nm.

Alternatively or additionally, Form S_{AcOH} can be characterized by having an FTIR spectrum comprising characteristic peaks at wavenumbers of 1693 ± 2 cm⁻¹, 1275 ± 2 cm⁻¹, 1098 ± 2 cm⁻¹, 832 ± 2 cm⁻¹ and 807 ± 2 cm⁻¹ when measured at room temperature with a diamond ATR cell. The FTIR spectrum of Form S_{AcOH} can be further characterized by having additional characteristic peaks at wavenumbers of 1508 ± 2 cm⁻¹, 1378 ± 2 cm⁻¹, 1219 ± 2 cm⁻¹, 1043 ± 2 cm⁻¹, 998 ± 2 cm⁻¹, 895 ± 2 and/or 626 ± 2 cm⁻¹ when measured at room temperature with a diamond ATR cell.

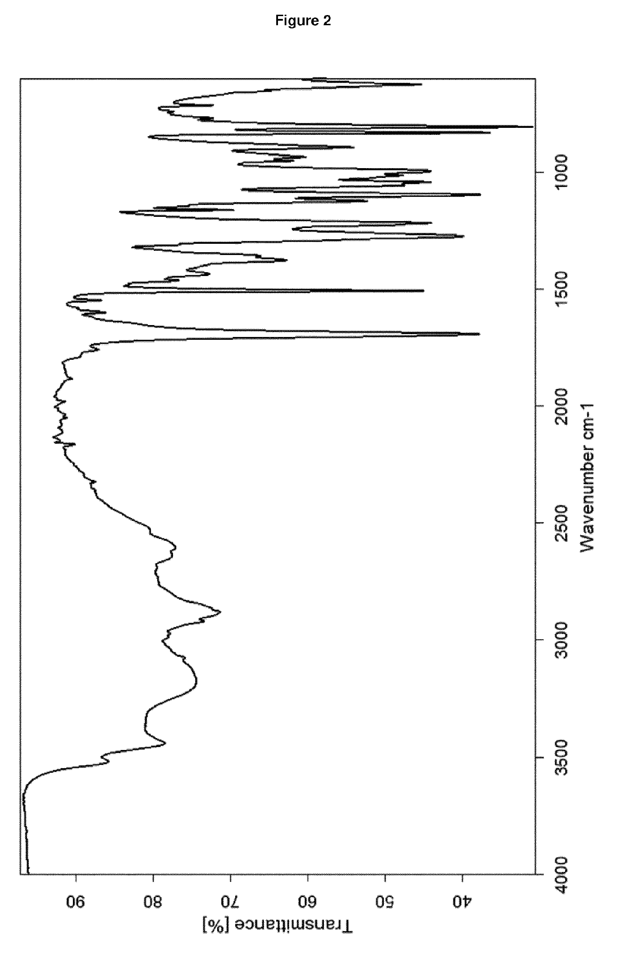

Alternatively or additionally, Form S_{AcOH} can be characterized by having an FTIR spectrum essentially the same as displayed in Figure 2 of the present invention when measured at room temperature with a diamond ATR cell.

Alternatively or additionally Form S_{AcOH} can be characterized by having a DSC curve comprising an endothermic peak with a peak maximum at about 75-76 °C when measured at a heating rate of 5 °C/min under nitrogen atmosphere.

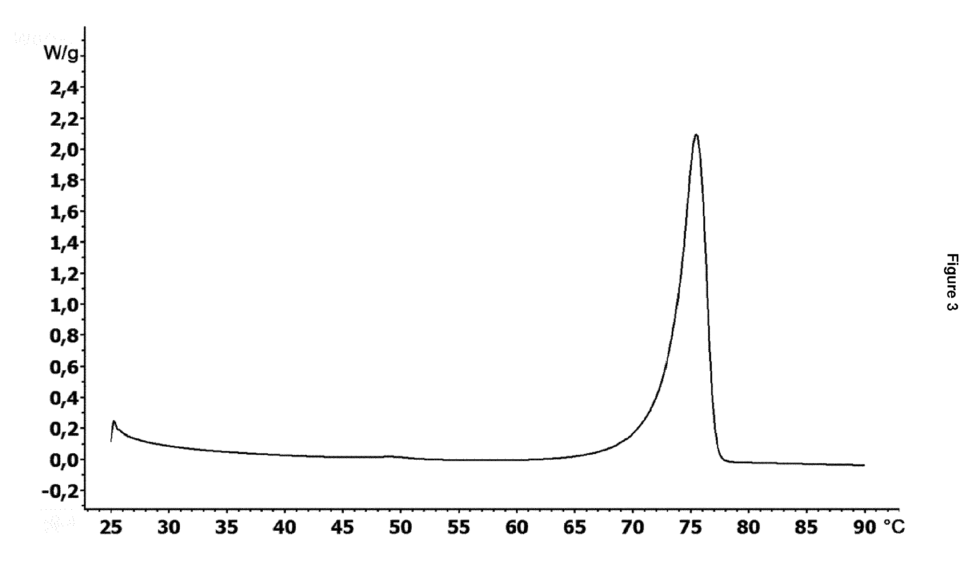

Alternatively or additionally Form S_{AcOH} can be characterized by having a DSC curve essentially the same as displayed in Figure 3 of the present invention when measured at a heating rate of 5 °C/min under nitrogen atmosphere.

In a second aspect, the present invention relates to a process for the preparation of Form S_{AcOH} comprising precipitating 1-(α-D-glucopyranosyl)-4-methyl-3-[5-(4-fluorophenyl)-2-thienylmethyl]benzene from a solvent comprising acetic acid, wherein the water content of the solvent is ≤ 10 volume%.

In a preferred embodiment, the present invention relates to a process for the preparation of Form S_{AcOH} comprising the steps of:
(i) forming a solution comprising 1-(β-D-glucopyranosyl)-4-methyl-3-[5-(4-fluorophenyl)-2-thienylmethyl]benzene and acetic acid, wherein the water content of the solution is ≤ 10 volume% and
(ii) precipitating Form S_{AcOH} from the solution obtained in step (i).

1-(β-D-glucopyranosyl)-4-methyl-3-[5-(4-fluorophenyl)-2-thienylmethyl]benzene starting material can be prepared in a similar manner as disclosed, for example, in Example 1 of WO 2005/012326 A1.

In a first step 1-(β-D-glucopyranosyl)-4-methyl-3-[5-(4-fluorophenyl)-2-thienylmethyl]benzene starting material is dissolved in a solvent comprising acetic acid, wherein the solution comprises ≤ 10 volume% water, preferably ≤ 5 volume% water and more preferably ≤ 2.5 volume% water. Most preferably glacial acetic acid is used as the solvent. The presence of water was found to delay or even prevent the crystallization of Form S_{AcOH} and to negatively influence the yield and the chemical purity of the final product (see Example 4 herein).

The obtained solution preferably comprises about 75 to 350 g, more preferably about 100 to 300 g and most preferably about 125 to 200 g 1-(β-D-glucopyranosyl)-4-methyl-3-[5-(4-fluorophenyl)-2-thienylmethyl]benzene per liter solvent. The solution is prepared at a temperature of about 20 to 60 °C, preferably of about 20 to 50 °C, more preferably of about 20 to 40 °C and most preferably the solution is prepared at a temperature of about 20 to 30 °C. The obtained solution may optionally be filtered in order to remove any undissolved particles. Optionally, the solution may be treated with charcoal before filtration.

The Form S_{AcOH} crystals are then allowed to precipitate. For that purpose the solution is kept at a temperature of about ≤ 40 °C, preferably of about ≤ 35 °C, more preferably of about ≤ 30 °C and most preferably at a temperature of about ≤ 25 °C. The solution is allowed to stand at the applied temperature or may be shaken, swirled or stirred, whereat stirring is most preferred. Usually Form S_{AcOH} crystals appear spontaneously, but precipitation may also be initiated by the addition of Form S_{AcOH} seed crystals, whereat the amount of seed crystals employed may be about 1 to 10 weight%, preferably about 1 to 7.5 weight%, more preferably about 1 to 5 weight% and most preferably about 1 to 2.5 weight% referred to the amount of 1-(β-D-glucopyranosyl)-4-methyl-3-[5-(4-fluorophenyl)-2-thienylmethyl]benzene starting material applied.

Once a homogenous suspension has been obtained the yield can be improved by antisolvent addition, whereat any liquid or mixture of liquids lowering the solubility of Form S_{AcOH} in the mother liquor may be used. Suitable antisolvents which may be employed are, for example, alkanes such as isooctane, n-heptane, n-hexane, cyclohexane and/or n-pentane. Most preferably n-heptane is employed as antisolvent. The amount of the antisolvent is chosen such that on the one hand the yield is satisfying and on the other hand the final product still shows the desired chemical purity. For example, when n-heptane is used the acetic acid/ antisolvent ratio (volume: volume) is about 1: 0.25-6, preferably 1: 0.25-4 more preferably 1: 0.25-2 and most preferably 1: 0.25-1 (see also Example 3 herein).

Subsequently, the crystals can be isolated by any conventional method such as filtration or centrifugation, whereat most preferably the crystals are collected by filtration. Optionally, an additional washing step may be applied, wherein the isolated Form S_{AcOH} crystals are contacted e.g. rinsed or slurried with a solvent comprising acetic acid, for example, with glacial acetic acid or an acetic acid/ antisolvent mixture as disclosed above.

Finally, the crystals are dried at a temperature of about ≤ 60 °C, preferably of about ≤ 50 °C, more preferably of about ≤ 40 °C and most preferably the crystals are dried at a temperature of about ≤ 30 °C for example at about room temperature. Drying can be performed under vacuum or at ambient pressure. Preferably, drying is performed by applying a vacuum of about ≤ 100 mbar, more preferably of about ≤ 50 mbar and most preferably of about ≤ 30 mbar. The crystals are preferably dried for about 6 to 72 hours, more preferably for about 12 to 48 hours and most preferably for about 18 to 24 hour.

Alternatively, Form S_{AcOH} crystals can be prepared by lyophilizing the solution obtained from step (i) of the second aspect of the present invention.

Therefore in a third aspect, the present invention relates to a process for the preparation of Form S_{AcOH} comprising a step of lyophilizing a solution comprising 1-(β-D-glucopyranosyl)-4-methyl-3-[5-(4-fluorophenyl)-2-thienylmethyl]benzene and acetic acid, wherein the water content of the solution is ≤ 10 volume%.

Several processes for the preparation of 1-(β-D-glucopyranosyl)-4-methyl-3-[5-(4-fluorophenyl)-2-thienylmethyl]benzene have been disclosed in the literature with little information about the chemical purity of the final products provided. As an exception, in WO 2008/069327 A1 the crystalline hemihydrate has been obtained from relatively impure 1-(β-D-glucopyranosyl)-4-methyl-3-[5-(4-fluorophenyl)-2-thienylmethyl]benzene. Example 2 of WO 2008/069327 A1 discloses crystallization of Form Hy0.5 from acetone/ water (1: 2 = volume: volume).

The present invention provides a novel crystalline form of 1-(β-D-glucopyranosyl)-4-methyl-3-[5-(4-fluorophenyl)-2-thienylmethyl]benzene, designated Form S_{AcOH} which is crystallized from acetic acid and is characterized by high chemical purity. Surprisingly, and contrary to the teaching of the prior art, the best yield and chemical purity of the final product are achieved by using acetic acid with water content of the solution being ≤ 10 volume%. As shown in Example 4 of the present invention the addition of water delays or even prevents crystallization of Form S_{AcOH} and, hence, negatively influences the yield and chemical purity of the final product. In terms of the yield and chemical purity, the best results are obtained by using glacial acetic acid.

For comparison, samples of 1-(β-D-glucopyranosyl)-4-methyl-3-[5-(4-fluorophenyl)-2-thienylmethyl]benzene having different grades of chemical purity have been employed in the process according to Example 2 of WO 2008/069327 A1 (production of Form Hy0.5) and in the process according to the present invention (production of Form S_{AcOH}), respectively. The chemical purity of the obtained Form S_{AcOH} was more than 2-fold higher compared to the chemical purity of the obtained Form Hy0.5, which was confirmed by HPLC at a wavelength of 220 nm. The results are summarized in Table 1.

**Table 1: Chemical purities of Form S_{AcOH} and Form Hy0.5 obtained from different starting materials**

| **Examples** | **Starting Material [area% HPLC]** | **Final Product [area% HPLC]** | **Form** |
|---|---|---|---|
| Example 1 | 98.7 | 99.8 (+1.1) | S_{AcOH} |
| Reference Example 1 | 98.7 | 99.1 (+0.4) | Hy0.5 |
| Example 2 | 97.2 | 99.4 (+2.2) | S_{AcOH} |
| Reference Example 2 | 97.2 | 98.2 (+1.0) | Hy0.5 |

Form S_{AcOH} can be used as a starting material for the preparation of further solid forms of 1-(β-D-glucopyranosyl)-4-methyl-3-[5-(4-fluorophenyl)-2-thienylmethyl]benzene, whereat the so obtained solid forms are characterized by having a similar high chemical purity. For example, Form S_{AcOH} can be converted to Form Hy0.5 by simply slurrying Form S_{AcOH} in water, whereat the chemical purity of the starting material is preserved (see Example 6 herein).

Therefore, Form S_{AcOH} is especially suitable for the purification of 1-(β-D-glucopyranosyl)-4-methyl-3-[5-(4-fluorophenyl)-2-thienylmethyl]benzene.

Making 1-(β-D-glucopyranosyl)-4-methyl-3-[5-(4-fluorophenyl)-2-thienylmethyl]benzene available in high chemical purity is highly desired as the presence of impurities is connected with certain toxicological risks. That's why impurities should be limited to the extent possible in order to ensure the highest quality, safety and efficacy of the final pharmaceutical drug product.

The following non-limiting examples are illustrative for the disclosure. Yields were calculated without considering residual solvents of starting materials or final products.

### EXPERIMENTAL PROCEDURES

PXRD was performed on an X'Pert PRO diffractometer (PANalytical, Almelo, The Netherlands) equipped with a theta/theta coupled goniometer in transmission geometry, programmable XYZ stage with well plate holder, Cu-Kα_{1,2} radiation source (wavelength 0.15419 nm) with a focusing mirror, a 0.5° divergence slit, a 0.02° soller slit collimator and a 0.5° anti-scattering slit on the incident beam side, a 2 mm anti-scattering slit, a 0.02° soller slit collimator, a Ni-filter and a solid state PIXcel detector on the diffracted beam side. The PXRDs were recorded at a tube voltage of 40 kV, tube current of 40 mA, applying a step size of 0.013° 2-Theta with 40 sec per step in the angular range of 2° to 40° 2-Theta. A typical precision of the 2-Theta values is in the range of ± 0.2° 2-Theta. Thus, the diffraction peak of Form S_{AcOH} that usually appears at 18.5° 2-Theta for example can appear between 18.3 and 18.7° 2-Theta on most X-ray diffractometers under standard conditions.

FTIR spectroscopy was performed on a MKII Golden Gate™ Single Reflection Diamond ATR (attenuated total reflection) cell with a Bruker Tensor 27 FTIR spectrometer with 4 cm⁻¹ resolution at room temperature. To record a spectrum a spatula tip of a sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 2 cm⁻¹. Thus, the infrared peak of Form S_{AcOH} that appears at 1693 cm⁻¹ can appear between 1691 and 1695 cm⁻¹ on most infrared spectrometers under standard conditions.

Differential scanning calorimetry (DSC) was performed on a Mettler Polymer DSC R instrument. Form S_{AcOH} (3.65 mg) was heated in a sealed 40 µL aluminum pan from 25 to 90 °C at a rate of 5 °C/min. Nitrogen (purge rate 50 mL/min) was used as purge gas.

### HPLC standard method:

| | | | |
|---|---|---|---|
| Column: | YMC-Pack Pro C18 150x4.6 mm I.D. S-5 µm, 12 nm | | |
| Column temperature: | 25 °C | | |
| Injection volume: | 3 µL | | |
| Eluent A: | 0.1 % formic acid/ water solution | | |
| Eluent B: | 0.1 % formic acid/ acetonitrile solution | | |
| Flow: | 1.2 mL/min | | |
| Pump gradient: | | | |

| | | | |
|---|---|---|---|
| | Time [min] | [%] B | |
| | 0.0 | 20 | |
| | 20.0 | 80 | |
| | 22.0 | 80 | |
| | 22.5 | 95 | |
| | 25.0 | 95 | |
| | 25.3 | 20 | |
| | 30.0 | 20 | |
| Detection: | 220 nm | | |
| Sample preparation: | The samples were dissolved in ACNL resulting in a concentration of about 1 mg/mL. | | |

### Preparation of 1-(β-D-glucopyranosyl)-4-methyl-3-[5-(4-fluorophenyl)-2-thienylmethyl]-benzene Form S_{AcOH} - Best Mode

### Example 1:

1.003 g 1-(β-D-glucopyranosyl)-4-methyl-3-[5-(4-fluorophenyl)-2-thienylmethyl]benzene hemihydrate (HPLC: 98.7 area%, prepared according to Example 2 of WO 2008/069327 A1) were dissolved in 7.5 mL glacial acetic acid at RT. The obtained clear solution was stirred at RT, whereat precipitation was observed within about 2 h. The suspension was further stirred for 2 h at RT before the crystals were collected by filtration, washed with 0.75 mL glacial acetic acid and dried at RT under vacuum (20-30 mbar) for 18 h to obtain 0.802 g of Form S_{AcOH}.

Yield: 80% of theory; PXRD: Form S_{AcOH}; FTIR: Form S_{AcOH}; HPLC: 99.8 area%;

**Table 2: PXRD peak list of Form S_{AcOH} prepared according to Example 1**

| PXRD peaks 2-Theta angles [± 0.2 °2-Theta] | | | |
|---|---|---|---|
| 4.8 | 15.9 | 19.3 | 22.4 |
| 6.7 | 16.9 | 19.6 | 24.2 |
| 7.9 | 17.1 | 20.0 | 24.8 |
| 9.6 | 17.5 | 20.2 | 25.1 |
| 9.7 | 17.7 | 20.7 | 26.5 |
| 12.7 | 18.5 | 21.2 | 29.9 |
| 14.6 | 19.0 | 21.5 | 30.3 |

**Table 3: FTIR peak list of Form S_{AcOH} prepared according to Example 1**

| FTIR peaks wavenumbers [± 2 cm⁻¹] | | | |
|---|---|---|---|
| 3519 | 1693 | 1162 | 895 |
| 3441 | 1508 | 1124 | 832 |
| 3176 | 1378 | 1098 | 807 |
| 2879 | 1275 | 1043 | 626 |
| 2605 | 1219 | 998 | |

### Example 2:

Example 1 was repeated using crude 1-(β-D-glucopyranosyl)-4-methyl-3-[5-(4-fluorophenyl)-2-thienylmethyl]benzene (HPLC: 97.2 area%, prepared in a similar manner as disclosed in Example 1 of WO 2005/012326 A1) as starting material.

PXRD: Form S_{AcOH}; HPLC: 99.4 area%

### Example 3: Preparation of 1-(β-D-glucopyranosyl)-4-methyl-3-[5-(4-fluorophenyl)-2-thienylmethyl]benzene Form S_{AcOH} - Yield improvement by antisolvent addition

About 1.000 g 1-(β-D-glucopyranosyl)-4-methyl-3-[5-(4-fluorophenyl)-2-thienylmethyl]benzene hemihydrate (HPLC: 98.7 area%, prepared according to Example 2 of WO 2008/069327 A1) were dissolved in 7.5 mL glacial acetic acid at RT. The obtained clear solution was stirred at RT, whereat precipitation was observed within about 2 h. After about 2 h of stirring n-heptane was added to the suspension in amounts according to Table 4 and the suspension was further stirred for 1 h at RT. Finally, the crystals were collected by filtration, washed with 0.75 mL of glacial acetic acid and dried at RT under vacuum (20-30 mbar) for 17 h.

**Table 4: Form S_{AcOH} preparation using variable amounts of n-heptane as antisolvent**

| **Example** | **n-Heptane [mL]** | **Yield [% of theory]** | **HPLC purity [area%]** | **PXRD** |
|---|---|---|---|---|
| 3a | 1.875 | 85 | 99.7 | S_{AcOH} |
| 3b | 3.750 | 92 | 99.7 | S_{AcOH} |
| 3c | 7.500 | 101 | 99.7 | S_{AcOH} |

### Example 4: Preparation of 1-(β-D-glucopyranosyl)-4-methyl-3-[5-(4-fluorophenyl)-2-thienylmethyl]benzene Form S_{AcOH} -Influence of water

About 1.000 g 1-(β-D-glucopyranosyl)-4-methyl-3-[5-(4-fluorophenyl)-2-thienylmethyl]benzene (HPLC: 99.6 area%, prepared in a similar manner as disclosed in Example 6 herein) were dissolved in 7.5 mL glacial acetic acid at RT. Water was added to the solution in amounts according to Table 5 and the solution was stirred at RT until a homogenous suspension was obtained. Where possible, the crystals were collected by filtration and dried at RT under vacuum (20-30 mbar) overnight.

**Table 5: Form S_{AcOH} preparation using variable amounts of water as cosolvent**

| **Example** | **Water [mL]** | **Precipitation** | **Yield [% of theory]** | **HPLC [area%]** | **PXRD** |
|---|---|---|---|---|---|
| 4a | 0 | after 20 min | 82 | 99.8 | S_{AcOH} |
| 4b | 0.75 | after stirring overnight | 48 | 99.0 | S_{AcOH} |
| 4c | 1.5 | no precipitation | - | - | - |

As can be seen from Table 5 the presence of water delays (see Example 4b) or even prevents (see Example 4c) the precipitation of Form S_{AcOH}. In addition, the presence of water negatively influences the yield as well as the chemical purity of the final product (see Example 4b).

### Example 5: Preparation of 1-(β-D-glucopyranosyl)-4-methyl-3-[5-(4-fluorophenyl)-2-thienylmethyl]benzene Form S_{AcOH} - Lyophilization

A solution of 1.006 g 1-(β-D-glucopyranosyl)-4-methyl-3-[5-(4-fluorophenyl)-2-thienylmethyl]benzene hemihydrate (prepared according to Example 2 of WO 2008/069327 A1) in 10 mL glacial acetic acid was freeze dried in order to obtain 1-(β-D-glucopyranosyl)-4-methyl-3-[5-(4-fluorophenyl)-2-thienylmethyl]benzene Form S_{AcOH} quantitatively.

Yield: quantitative; PXRD: Form S_{AcOH}

### Example 6: Preparation of Form Hy0.5 from Form S_{AcOH}

0.504 g 1-(β-D-glucopyranosyl)-4-methyl-3-[5-(4-fluorophenyl)-2-thienylmethyl]benzene Form S_{AcOH} (HPLC: 99.7 area%, obtained from Example 3a herein) were slurried in 50 mL water for 4 h using a magnetic stirrer. Subsequently, the crystals were collected by filtration and dried at room temperature under vacuum (20-30 mbar) for 17 h to obtain 0.391 g Form Hy0.5.

Yield: 78% of theory; PXRD: Form Hy0.5; HPLC: 99.5 area%

### Preparation of 1-(β-D-glucopyranosyl)-4-methyl-3-[5-(4-fluorophenyl)-2-thienylmethyl]-benzene hemihydrate (Form Hy0.5) according to the procedure disclosed in Example 2 of WO 2008/069327 A1

### Reference Example 1:

0.498 g 1-(β-D-glucopyranosyl)-4-methyl-3-[5-(4-fluorophenyl)-2-thienylmethyl]benzene hemihydrate (HPLC: 98.7 area%, prepared according to Example 2 of WO 2008/069327 A1) were dissolved in 4.6 mL acetone and thereto were added 9.2 mL water and a spatula tip of Form Hy0.5 seed crystals. The mixture was stirred at room temperature for 24 h before the precipitate was collected by filtration washed with 9.2 mL acetone/ water (1: 4 = volume: volume) and dried at room temperature under vacuum (20-30 mbar) to give 0.447 g Form Hy0.5.

Yield: 90% of theory; PXRD: Form Hy0.5; HPLC: 99.1 area%

### Reference Example 2:

1.625 g crude 1-(β-D-glucopyranosyl)-4-methyl-3-[5-(4-fluorophenyl)-2-thienylmethyl]benzene (HPLC: 97.2 area%, prepared in a similar manner as disclosed in Example 1 of WO 2005/012326 A1) were dissolved in 15 mL acetone and thereto were added 30 mL water and a spatula tip of Form Hy0.5 seed crystals. The mixture was stirred at room temperature for 22 h before the precipitate was collected by filtration washed with 30 mL acetone/ water (1: 4 = volume: volume) and dried at room temperature under vacuum to give 1.188 g Form Hy0.5.

Yield: 73% of theory; PXRD: Form Hy0.5; HPLC: 98.2 area%

## Claims

1. A crystalline form of a compound of formula (I) 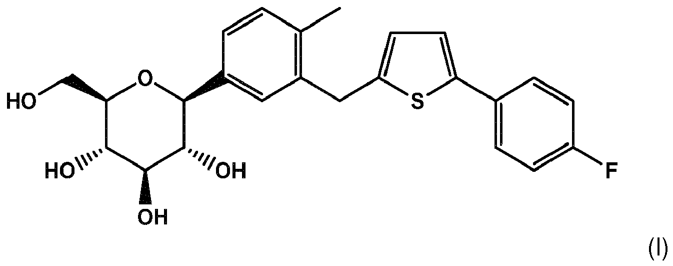 **characterized by** a powder X-ray diffractogram comprising characteristic peaks, double peaks and/or multiple peaks at 2-Theta angles of 4.8 ± 0.2°, 7.9 ± 0.2°, 14.6 ± 0.2°, 18.5 ± 0.2° and 21.5 ± 0.2° when measured at 15 to 25 °C with Cu-Kα_{1,2} radiation having a wavelength of 0.15419 nm.

2. The crystalline form according to claim 1 **characterized by** a FTIR spectrum comprising characteristic peaks at wavenumbers of 1693 ± 2 cm⁻¹, 1275 ± 2 cm⁻¹, 1098 ± 2 cm⁻¹, 832 ± 2 cm⁻¹ and 807 ± 2 cm⁻¹ when measured at 15 to 25 °C with a diamond ATR cell.

3. The crystalline form according to any one of claims 1 and 2 **characterized by** a DSC curve comprising an endotherm with a peak maximum at 75 to 76 °C when measured at a heating rate of 5 °C/min under nitrogen atmosphere.

4. A process for the preparation of the crystalline form according to any one of claims 1 to 3 comprising precipitating 1-(β-D-glucopyranosyl)-4-methyl-3-[5-(4-fluorophenyl)-2-thienylmethyl]benzene from a solvent comprising acetic acid, wherein the water content of the solvent is ≤ 10 volume%.

5. The process according to claim 4, said process comprising the steps of:
(i) forming a solution comprising 1-(β-D-glucopyranosyl)-4-methyl-3-[5-(4-fluorophenyl)-2-thienylmethyl]benzene and acetic acid, wherein the water content of the solution is ≤ 10 volume%, and
(ii) precipitating the crystalline form according to any one of the claims 1 to 3 from the solution obtained in step (i).

6. The process according to claim 5, wherein an antisolvent is added to the suspension obtained in step (ii).

7. The process according to claim 6, wherein the antisolvent is n-heptane.

8. The process according to any of claims 5 to 7, wherein the crystals obtained are further isolated by filtration or centrifugation.

9. The process according to claim 8, wherein the isolated crystals are further dried at a temperature of ≤ 60 °C.

10. A process for the preparation of the crystalline form according to any one of claims 1 to 3 comprising a step of lyophilizing a solution comprising 1-(β-D-glucopyranosyl)-4-methyl-3-[5-(4-fluorophenyl)-2-thienylmethyl]benzene and acetic acid, whereat the water content of the solution is ≤ 10 volume%.

11. The process according to any one of claims 4 to 10, wherein the solvent comprising acetic acid is glacial acetic acid.

12. Use of a solvent comprising acetic acid for the purification of 1-(β-D-glucopyranosyl)-4-methyl-3-[5-(4-fluorophenyl)-2-thienylmethyl]benzene, wherein the water content of the solvent is ≤ 10 volume%.

13. Use according to claim 12, wherein the solvent comprising acetic acid is glacial acetic acid.

14. Use of the crystalline form according to any one of claims 1 to 3 for the purification of 1-(β-D-glucopyranosyl)-4-methyl-3-[5-(4-fluorophenyl)-2-thienylmethyl]benzene.
